# EUROPÄISCHE PATENTANMELDUNG

(11) **EP 1 366 739 A1**
(43) Veröffentlichungstag der Anmeldung: **03.12.2003**
(21) Anmeldenummer: 03011974.7
(22) Anmeldetag: 28.05.2003
(51) Int. Cl.: A61K 7/00, A61K 7/06, A61K 7/075, A61K 7/08

(54) **Pflegendes Haarshampoo**

(30) Priorität: 31.05.2002 DE 10224025
(71) Anmelder: Beiersdorf AG, 20245 Hamburg (DE)
(72) Erfinder: Argembeaux, Horst, 21465 Wentorf (DE); Primmel, Bettina, 20146 Hamburg (DE); Kaiser, Elke, 22769 Hamburg (DE)

(57) **Zusammenfassung**

Kosmetische Zubereitung enthaltend
a) ein oder mehrere Guar-Hydroxypropyltrimethylammoniumchloride,
b) ein oder mehrere Fettalkohole und/oder deren Derivate,
c) ein oder mehrere Tenside,
neben gegebenenfalls weiteren kosmetischen Wirk-, Hilfs-, und Zusatzstoffen.

## Beschreibung

Die vorliegende Erfindung betrifft eine kosmetische Zubereitung enthaltend ein oder mehrere Guar-Hydroxypropyltrimethylammoniumchloride, ein oder mehrere Fettalkohole und/oder deren Derivate sowie ein oder mehrere Tenside.

Bei der Körperpflege des Menschen spielt die Haarwäsche eine zentrale Rolle. Die Reinigung der Haare und der Kopfhaut von körpereigenem Fett, Hautabschilferungen, Schmutz und Gerüchen entspricht einem Grundbedürfnis des Menschen.

Zur Befriedigung dieses Bedürfnisses stand dem Menschen bis ins 20.Jahrhundert hinein allein Seife zur Verfügung, die aufgrund ihres alkalischen pH-Wertes für die Kopfhaut und die Augenschleihäute wenig verträglich war und häufig Ablagerungen von Kalkseifen im Haar zurück ließ. In den dreißiger Jahren des 20.Jahrhunderts erblickte das erste alkylsulfathaltige Shampoo das Licht der Welt. Seit Mitte der sechziger Jahre erobern Alkylethersulfate und andere Tenside den Shampoomarkt. Mit ihnen können die Nachteile von seifenhaltigen Zubereitungen vermieden werden. Heutzutage müssen moderne Shampoos das Haar nicht nur reinigen und gut verträglich sein. Vielmehr sollen sie das Haar auch pflegen und seine Frisierbarkeit und optische Attraktivität erhöhen.

Haarshampoos enthalten eine Vielzahl unterschiedlicher Komponenten um den einzelnen Anforderungen an das Produkt gerecht zu werden:

Die Reinigungskraft der Shampoos wird bewirkt durch die Anwesenheit von anionischen, amphoteren und nichtionischen Tensiden als oberflächenaktive Verbindungen in den Zubereitungen. Tenside sorgen darüber hinaus für das Schaumvermögen der Haarreinigungsmittel. Wichtig bei der Auswahl der Tenside ist darüber hinaus ihre Unempfindlichkeit gegenüber der Wasserhärte, ihre biologische Abbaubarkeit, ihre Verträglichkeit mit anderen Komponenten der Zubereitung sowie ihr Preis. Ein viel verwendetes Shampootensid ist beispielsweise Alkylethersulfat.

Darüber hinaus enthalten Shampoos eine Reihe von Konsistenzregulatoren, die der Zubereitung die gewünschte Viskosität verleihen. Diese Verdicker bewirken eine Vergrößerung der Tensidmicellen bzw. eine Quellung der Wasserphase der Zubereitung. Verdicker können aus chemisch sehr unterschiedlichen Stoffklassen gewählt werden. So werden u.a. Elektrolyte (z.B. Natriumchlorid), Alkanolamide (z.B. Fettsäure-Monoethanolamide), niedrig ethoxylierte Fettalkohole (z.B. Diethylenglycolmonolaurylether), hochethoxylierte Ether, Ester und Diester sowie polymere Verdicker eingesetzt. Zu den polymeren Verdickern zählen beispielsweise Celluloseether. Darüber hinaus finden auch Polyacrylate und Hydrokolloide als Verdicker Verwendung. Polymere Verdicker haben den großen Vorteil, dass die durch sie erzeugte Viskosität weitgehend temperaturunabhängig ist.

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. Hierzu zählen neben UV-Absorbern, Vitaminen oder Pflanzenextrakten auch sogenannte Haarkonditionierer (engl. conditioner), welche das Haar pflegen und seine Kämmbarkeit und seinen Griff verbessern sowie seinen Glanz erhöhen. Konditionierer ziehen, im Gegensatz zu den meisten anderen Bestandteilen von Shampoos, auf das Haar auf und verbleiben dort nach dem Spülen. Sie lagern sich aufgrund ihres Molekülaufbaus an die Schadstellen der Cuticula des Haares und glätten das Haar. Dadurch wird das Haar weniger rauh und spröde, die Frisur bekommt deutlich mehr Glanz und läßt sich leichter kämmen. Auch wird das Haar weniger empfindlich für eine elektrostatische Aufladung. Die wichtigsten haarkonditionierenden Substanzen, in der Regel kationische Polymere, stellen die polymeren quatären Ammoniumverbindungen dar. Auch können kationische Cellulosederivate und Polysaccharide eingesetzt werden. Weiterhin werden auch Silikonverbindungen zur Konditionierung eingesetzt.
Ein Nachteil am Stande der Technik besteht in dem Umstand, dass durch häufiges Waschen oder chemische Behandlung (Bleichen, Färben) das Haar stark geschädigt wird. Insbesondere führt die Entfernung/Zerstörung von natürlich im Haar vorkommenden Fettkomponenten zu einem Verlust an Glätte, Geschmeidigkeit und Glanz. Das Haar erscheint dann trocken und spröde. Um diese Schäden ausgleichen zu können, werden den Haarpflegeprodukten in der Regel große Mengen an kationischen Polymeren als Haarkonditionierern zugesetzt. Um den Fettverlust auszugleichen, werden derartigen Produkten häufig Silikonverbindungen oder weitere hydrophobe Konditionierer zugesetzt. Diese nach dem Stande der Technik hergestellten Produkte haben jedoch eine Reihe von Nachteilen:
■ Es lagern sich bei jeder Anwendung herkömlicher Produkte zusätzliche Schichten an Konditionierer auf dem Haar ab (engl. build-up-effect). Das Haar wird durch die großen Mengen an Konditionierern beschwert und bekommt ein strähniges Aussehen.
■ Die Rückfettung des Haares ist in der Regel mangelhaft.
■ Die in der Regel schäumenden Produkte (Shampoos) haben bei einer hohen Einsatzkonzentration von Pflegestoffen eine schlechte Schaumsensorik. Der Schaum fühlt sich glitschig an.
■ In der Regel werden "unnatürliche", synthetisch hergestellte Konditionierer eingesetzt, deren physiologische Verträglichkeit bei empfindlichen Personen nicht immer gewährleistet werden kann, deren biologische Abbaubarkeit zu wünschen übrig läßt und die sich beim Verbraucher zunehmend geringerer Akzeptanz erfreuen.

Es war daher die Aufgabe der vorliegenden Erfindung die Mängel des Standes der Technik zu beseitigen oder zumindest zu lindern und gut verträgliche Haarpflegeprodukte zu entwickeln, welche selbst stark geschädigtes Haar wieder zu neuem Glanz, Fülle und Spannkraft verhelfen.

Überraschend gelöst wird die Aufgabe durch eine kosmetische Zubereitung enthaltend
a) ein oder mehrere mehrere Guar-Hydroxypropyltrimethylammoniumchloride,
b) ein oder mehrere Fettalkohole und/oder deren Derivate,
c) ein oder mehrere Tenside,
neben gegebenenfalls weiteren kosmetischen Wirk-, Hilfs-, und Zusatzstoffen.

Die erfindungsgemäßen Zubereitungen verleihen selbst stark geschädigten Haar wieder neuen, seidigen Glanz. Das mit diesen Zubereitungen gepflegte Haar weist jeweils sowohl im nassen, wie auch im trockenen Zustand eine hervorragende Kämmbarkeit sowie einen guten Griff auf. Aufgeschäumte erfindungsgemäße Zubereitungen haben darüber hinaus eine angenehme Schaumsensorik, die von den Anwendern als cremig oder samtig beschrieben wird.

Zwar beschreiben sowohl die EP 86 52 272 konditionierende Shampoos mit einer Mischung aus Tensiden, Fettalkohol(-derivaten) und Polyolen als auch die WO 200100149 kosmetische Zubereitungen mit Fettalkoholen und Tensiden, doch konnten diese Schriften nicht den Weg zur vorliegenden Erfindung weisen.

Die erfindungsgemäß vorteilhaften kosmetischen Zubereitungen sind dadurch gekennzeichnet, dass das oder die Guar-Hydroxypropyltrimethylammoniumchloride eine Ladungsdichte von 0,4 bis 1,0 meq/g sowie ein Molekulargewicht von 100000 bis 1800000 aufweisen. Erfindungsgemäß bevorzugte Guar-Hydroxypropyltrimethylammoniumchloride stammen aus der Jaguar-Serie, erfindungsgemäß besonders bevorzugt ist das Produkt Jaguar Excel der Firma Rhodia.

Es ist erfindungsgemäß vorteilhaft ein oder mehrere mehrere Guar-Hydroxypropyltrimethylammoniumchloride in einer Konzentration von 0,01 bis 10 Gewichts-%, bevorzugt in einer Konzentration von 0,01 bis 2 Gewichts-% und ganz besonders bevorzugt in einer Konzentration 0,01 von bis 0,5 Gewichts-%, jeweils bezogen auf das Gesamtgewicht der Zubereitung einzusetzen.

Vorteilhaft im Sinne der vorliegenden Erfindung sind Fettalkohole und/oder deren Derivate welche 8 bis 20 Kohlenstoffatome pro Molekül aufweisen.

Ferner ist es erfindungsgemäß vorteilhaft, wenn die Fettalkohole und/oder deren Derivate eine Kohlenstoffanzahl von C8-C20 aufweisen.

Es ist erfindungsgemäß vorteilhaft, wenn die Konzentration ein oder mehrerer Fettalkohole und/oder deren Derivate 0,01 bis 5 Gewichts-%, bevorzugt 0,01 bis 2 Gewichts-% und ganz besonders bevorzugt von 0,01 bis 1,5 Gewichts-%, jeweils bezogen auf das Gesamtgewicht der Zubereitung beträgt.

Es ist ferner erfindungsgemäß vorteilhaft, wenn das Verhältnis von Guar-Hydroxypropyltrimethylammoniumchloriden zu Fettalkoholen und/oder deren Derivaten in den erfindungsgemäßen Zubereitungen von 1:10 bis 10:1 und besonders bevorzugt von 1:5 bis 5:1 beträgt.

Die erfindungsgemäßen kosmetischen Zubereitungen enthalten Tenside. Vorteilhafte waschaktive anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroyisarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

Vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole,
■ ethoxylierte Amine,
insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß besonders bevorzugt, wenn als Tenside anionische, amphotere und/oder nichtionische Tenside eingesetzt werden wobei es ganz besonders bevorzugt ist, wenn als Tenside Natriumlaurylethersulfat, Cocoamidopropylbetain und/oder Natriumlauroylsulfosuccinat eingesetzt werden.

Eine erfindungsgemäß besonders vorteilhafte Ausführungsform der vorliegenden Erfindung stellt insbesondere die Kombination der Tenside von Natriumlaurylethersulfat mit Cocoamidopropylbetain dar.

Ferner können Polysorbate als waschaktive Agentien erfindungsgemäß vorteilhaft in die Emulsion eingearbeitet werden.

Es ist erfindungsgemäß von Vorteil, wenn ein oder mehrere Tenside in einer Konzentration von 1 bis 25 Gewichts-%, bevorzugt in einer Konzentration 7 von 18 bis Gewichts-% und ganz besonders bevorzugt in einer Konzentration von 7 bis 15 Gewichts-%, jeweils bezogen auf das Gesamtgewicht der Zubereitung eingesetzt wird.

Vorteilhafte Ausführungsformen der erfindungsgemäßen kosmetischen Zubereitung sind auch dadurch gekennzeichnet, dass sie als weitere Bestandteile Trübungsmittel und/oder Perlglanzmittel enthalten. Als Trübungsmittel werden dabei erfindungsgemäß Substanzen und Substanzgemische verstanden, welche der Zubereitung ein trübes emulsionsartiges Aussehen verleihen.
Als Perlglanzmittel werden dabei erfindungsgemäß Substanzen und Substanzgemische verstanden, welche der Zubereitung ein opaleszierendes Aussehen verleihen.

Erfindungsgemäß vorteilhaft ist es auch Mischungen aus Trübungs- und Perlglanzmittel einzusetzen.
Erfindungsgemäß vorteilhafte Trübungsmittel/Perlglanzmittel bzw. Mischungen sind unter anderem:
■ PEG-3 Distearat (z.B. CUTINA TS der Firma Cognis),
■ eine Kombination aus Glycoldistearat, Glycerin, Laureth-4 und Cocamidopropylbetain (z.B. Euperlan PK 3000 und Euperlan PK 4000 der Firma Cognis),
■ eine Kombination aus Glycoldistearat, Cocosglucosiden, Glyceryloleat und Glycerylstearat (z.B. Lamesoft TM Benz der Firma Cognis).
■ Styrol/Acrylat Copolymere (z.B. Acusol OP 301 von Rohm & Haas)

Ferner ist es erfindungsgemäß von Vorteil, wenn in den erfindungsgemäßen Zubereitungen Verdicker eingesetzt werden. Diese können beispielsweise vorteilhaft aus weiteren Verbindungen der Gruppe der Gummen gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate als erfindungsgemäß vorteilhafte Verdicker.

Unter den Cellulosederivaten befinden sich z.B. Methylcellulose, Carboxymethylcellulose, Hydroxyethylcellulose, Hydroxypropylmethylcellulose als erfindungsgemäß vorteilhafte Verdicker.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form als Verdicker verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Noveon (Carbopol 980, 981, 1382, 5984, 2984, ETD 2001, ETD 2020, ETD 2050 oder Pemulen TR1 & TR2).

Unter den Polymeren befinden sich z.B. Polyacrylamide (Seppigel 305), Polyvinylalkohole, PVP, PVP / VA Copolymere, Polyglycole.

Vorteilhaft, wenn auch nicht zwingend, können die erfindungsgemäßen Zubereitungen Konservierungsmittel enthalten.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Diese Liste der vorteilhaften Konservierungsmittel soll keineswegs limitierend sein. Vielmehr sind alle für Kosmetika oder Lebensmittel zugelassenden Konservierungsmittel vorteilhaft im Sinne der vorliegenden Erfindung.

Die wässrige Phase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon], beispielsweise Carbopole der Typen 980, 981, 1382, 2984, 5984, ETD 2020, ETD 2050, Ultrez 10, jeweils einzeln oder in Kombination.

Die kosmetischen Zubereitungen gemäß der Erfindung können kosmetische Hilfsstoffe enthalten, wie sie üblicherweise in solchen Zubereitungen verwendet werden, z. B. Konservierungshelfer, Komplexbildner, Antioxidantien, Puffer, Lösungsvermittler, Dispergiermittel, Bakterizide, Parfüme, Substanzen zum Verhindern oder Steigern des Schäumens, Farbstoffe, Pigmente, die eine färbende Wirkung haben, Verdickungsmittel, Perlglanzpigmente, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Antischuppenwirkstoffe, Pflanzenextrakte, Vitamine, Wirkstoffe.

Insbesondere ist es erfindungsgemäß von Vorteil der erfindungsgemäßen Zubereitung Vitamine, Pflanzenextrakte und UV-Lichtschutzfilter zuzusetzen. So ist beispielsweise der Zusatz von Calcium-Vitamin-Komplexen erfindungsgemäß besonders vorteilhaft.

Ferner ist es im Sinne der vorliegenden Erfindung, der erfindungsgemäßen Zubereitung Perlglanzpigmente, Glimmer und/oder Effektpigmente zuzusetzen, um die Zubereitung optisch attraktiver zu gestalten.

Die Zubereitungen im Sinne der vorliegenden Erfindung können bevorzugt in Form von W/O-, O/W-, W/O/W- oder O/W/O-Emulsionen vorliegen. Solche Formulierungen können vorzugsweise auch als Mikroemulsion (z. B. eine PIT-Emulsion) oder als Feststoff-Emulsionen (d. h. eine Emulsion, welche durch Feststoffe stabilisiert ist, z. B. eine Pickering-Emulsion) vorliegen

Die erfindungsgemäßen Zubereitungen stellen in jeglicher Hinsicht überaus befriedigende Präparate dar, welche nicht auf die in dieser Schrift erwähnte Rohstoffauswahl begrenzt sind.

Es ist erfindungsgemäß vorteilhaft, wenn die erfindungsgemäße kosmetische Zubereitung in einer Flasche, Quetschflasche, Pumpspray-, oder Aerosoldose aufbewahrt und aus dieser heraus angewendet werden. Entsprechend sind auch Flaschen, Quetschflaschen, Pumpspray-, oder Aerosoldosen, welche eine erfindungsgemäße Zubereitung enthalten, erfindungsgemäß.

Die erfindungsgemäßen Zubereitungen werden vorteilhaft zur Pflege des Haars, insbesondere des Kopfhaares, eingesetzt.

Nicht zuletzt ist die Verwendung einer erfindungsgemäßen kosmetische Zubereitung als Haarwaschmittel (Shampoo). und/oder Haarkonditionierer, d.h. als Mittel zum Konditionieren des Haares, erfindungsgemäß.

Die folgenden Beispiele, in welchen Waschpräparate zur Haarpflege beschrieben werden, sollen die erfindungsgemäßen Zusammensetzungen erläutern, ohne dass aber beabsichtigt ist, die Erfindung auf diese Beispiele zu beschränken. Die Zahlenwerte in den Beispielen bedeuten Gewichtsprozente, bezogen auf das Gesamtgewicht der jeweiligen Zubereitungen.

### Beispielrezepturen

| | **1** | **2** | **3** | **4** | **5** |
|---|---|---|---|---|---|
| Natrium Laurylethersulfat | 9 | 9 | 9 | 9 | 11 |
| Cocamidopropyl Betain | 2,5 | 2,5 | 2,5 | 2,5 | 5,0 |
| Natrium Lauroylsulfosuccinat | 3 | 3 | 3 | 3 | - |
| Natrium Carbomer | 0,3 | 0,3 | 0,3 | 0,3 | 0,6 |
| Guar Hydroxypropyl Trimonium Chlorid (Jaguar Excel von Rhodia) | 0,3 | 0,3 | 0,3 | 0,3 | 0,4 |
| C16/C18 Fettalkohol (Jaguar Excel von Rhodia) | 0,5 | 0,5 | 0,5 | 0,5 | 1,0 |
| PEG-3 Distearate (CUTINA TS von Cognis) | 2,0 | - | - | - | 1,5 |
| Glycol Distearat + Glycerin + Laureth-4 + Cocamidopropyl Betain (Euperlan PK 3000 OK von Cognis) | - | 2,5 | - | - | - |
| Glycol Distearat + Laureth-4 + Cocamidopropyl Betain (Euperlan PK 4000 von Cognis) | - | - | 2,5 | - | - |
| Glycol Distearat + Coco Glucoside + Glyceryl Oleate + Glyceryl Stearat (Lamesoft TM Benz von Cognis) | - | - | - | 2,5 | - |
| PEG-40 hydriertes Rizinusöl | 0,2 | 0,2 | 0,2 | 0,2 | 0,2 |
| Natrium Salicylat | 0,4 | 0,4 | 0,4 | 0,4 | 0,4 |
| Natrium Benzoat | 0,4 | 0,4 | 0,4 | 0,4 | 0,4 |
| Parfüm | q.s. | q.s. | q.s. | q.s. | q.s. |
| Wasser | ad 100 | ad 100 | ad 100 | ad 100 | ad 100 |

## Patentansprüche

1. Kosmetische Zubereitung enthaltend
a) ein oder mehrere Guar-Hydroxypropyltrimethylammoniumchloride,
b) ein oder mehrere Fettalkohole und/oder deren Derivate,
c) ein oder mehrere Tenside,
neben gegebenenfalls weiteren kosmetischen Wirk-, Hilfs-, und Zusatzstoffen.

2. Kosmetische Zubereitung nach Anspruch 1, **dadurch gekennzeichnet, dass** das oder die Guar-Hydroxypropyltrimethylammoniumchloride eine Ladungsdichte von 0,4 bis 1,0 meq/g sowie ein Molekulargewicht von 100000 bis 1800000 aufweisen.

3. Kosmetische Zubereitung nach einem der Ansprüche 1 oder 2, **dadurch gekennzeichnet, dass** als Fettalkohole und/oder deren Derivate Verbindungen mit 8 bis 20 Kohlenstoffatomen pro Molekül eingesetzt werden.

4. Kosmetische Zubereitung nach einem der Ansprüche 1 bis 3, **dadurch gekennzeichnet, dass** die Fettalkohole und/oder deren Derivate eine Kohlenstoffanzahl von C8-C20 aufweisen.

5. Kosmetische Zubereitung nach einem der Ansprüche 1 bis 4, **dadurch gekennzeichnet, dass** sie
a) ein oder mehrere mehrere Guar-Hydroxypropyltrimethylammoniumchloride in einer Konzentration von 0,01 bis 10 Gewichts-%,
b) ein oder mehrere Fettalkohole und/oder deren Derivate in einer Konzentration von 0,01 bis 5 Gewichts-%,
c) ein oder mehrere Tenside in einer Konzentration von 1 bis 25 Gewichts-% jeweils bezogen auf das Gesamtgewicht der Zubereitung enthalten.

6. Kosmetische Zubereitung nach einem der Ansprüche 1 bis 5, **dadurch gekennzeichnet, dass** als Tenside anionische, amphotere und/oder nichtionische Tenside und ganz besonders bevorzugt, Natriumlaurylethersulfat, Cocoamidopropylbetain und/oder Natriumlauroylsulfosuccinat eingesetzt werden.

7. Kosmetische Zubereitung nach einem der Ansprüche 1 bis 6, **dadurch gekennzeichnet, dass** das Verhältnis von Guar-Hydroxypropyitrimethylammoniumchloriden zu Fettalkoholen und/oder deren Derivaten von : bis : beträgt.

8. Kosmetische Zubereitung nach einem der Ansprüche 1 bis 7, **dadurch gekennzeichnet, dass** sie als weitere Bestandteile Trübungsmittel und/oder Perlglanzmittel und/oder Verdicker enthält.

9. Verwendung der kosmetischen Zubereitung nach einem der vorhergehenden Ansprüche als Haarwaschmittel (Shampoo).
